# EUROPEAN PATENT SPECIFICATION

(11) **EP 1 718 225 B1**
(45) Date of publication and mention of the grant of the patent: **08.04.2009**
(21) Application number: 05708365.1
(22) Date of filing: 15.02.2005
(51) Int. Cl.: A61B 17/32, A61B 17/22, A61B 17/00

(54) **NOZZLE FOR USE IN GENERATING A PRESSURE JET OF LIQUID**
DÜSE ZUR VERWENDUNG BEI DER ERZEUGUNG EINES DRUCKSTRAHLS VON FLÜSSIGKEIT
BUSE PERMETTANT DE PRODUIRE UN JET DE LIQUIDE SOUS PRESSION

(30) Priority: 17.02.2004 GB 0403512
(43) Date of publication of application: 08.11.2006
(73) Proprietor: Eschmann Holdings Limited, Lancing, West Sussex BN15 8TJ (GB)
(72) Inventor: GONON, Bertrand, F-69370 Ternay (FR)
(74) Representative: Peel, James Peter
(86) International application number: PCT/GB2005/000553
(87) International publication number: WO 2005/079681

(56) References cited:
- DE-A1- 3 421 390
- DE-A1- 19 506 002
- DE-A1- 19 904 640
- US-A- 4 898 574
- US-A- 6 110 164
- US-A1- 2003 125 660
- US-B1- 6 423 027

## Description

The present invention relates to a nozzle for use in the generation of a high-pressure liquid jet and to a surgical dissection instrument using a high-pressure liquid jet, comprising a handpiece provided with such a nozzle.

There are numerous known surgical dissection instruments using a high-pressure liquid jet, also known in the art as a lancet. Some of these instruments are relatively complex, and so are quite costly, which means that it is not commercially acceptable for them to be disposable so that are used only once. However, the sterilisation requirements which are imposed on this type of reusable instrument, and the problems posed when reusing it, particularly as regards the risks of contagion, make it virtually obligatory to use it once only.

To solve this problem, the applicant has previously proposed, in EP-A-0840626 and its equivalent US-A-6066150, a surgical dissection instrument using a high-pressure liquid jet having a handpiece which was disposable, yet reliably fulfilled the functions of the handpiece, namely to control the supply and stop the supply of high-pressure liquid, to lock the liquid supply control in the stop position, and to provide a suction function for evacuating fluid and body matter away from the dissection site in the patient's body. The surgical dissection instrument was specially configured as a disposable device, so that it could be used only once in an economically acceptable manner.

The handpiece disclosed in EP-A-0840626 is fitted with a nozzle for generating the jet of high-pressure liquid. As shown in Figure 1. in this known construction the nozzle is a rigid cylindrical metal tube 1 having a narrow bore 2 extending therealong and provided at its distal end with a sapphire body (or die) 3 having an orifice 4 therein of approximately 0.1mm diameter. The sapphire body 3 is mounted in a preformed setting 5 in the end of the tube 1, and then the end of the tube 1 is deformed to secure the sapphire body 3 in the setting 5. A high pressure jet is emitted from the orifice 4 when pressurized liquid is fed down the narrow bore. The proximal end 6 of the nozzle is securely fitted to an end of a conduit in the handpiece through which the liquid is fed, so that the nozzle is not separated from the handpiece under the action of the fluid pressure during operation. The proximal end 6 is threaded and is fitted to a plastic conduit in the handpiece. The sapphire body 3 is secured within the end of the metal tube 1. so that the sapphire body 3 is also not separated from the nozzle during operation under the action of the fluid pressure. Either eventuality would obviously be very dangerous for the patient, and also the surgical team. Accordingly, the nozzle is permanently fitted to the handpiece, for disposal together with the handpiece after a single use.

A sapphire body has been used in the art because of the need to ensure the safety and integrity of the surgical dissection instrument. The sapphire crystal permits an orifice of precisely controlled diameter to be accurately provided, which resists the very high liquid pressures and is highly resistant to any abrasion in use. The sapphire crystal can readily be securely bonded to a metal nozzle, so as to ensure that the nozzle can safely be subjected to high liquid pressures. However, the use of a sapphire body adds expense and complexity to the manufacture of what is a disposable item. If it is not completely ensured that the sapphire body is permanently and securely bonded into the nozzle, the safety of the device is compromised. The correct and secure disposition of the sapphire body into the setting in the metal tube is problematic and expensive. The metal tube also provides high resistance to high liquid pressures, but can be difficult and expensive to manufacture, and can be difficult to fit to the plastic conduit.

In a later development of the nozzle disclosed in EP-A-0840626, it has also been proposed in the art to use, instead of a sapphire crystal, a metal body which is securely bonded into the end of the nozzle. The metal body has lower cost than the sapphire body. The metal body is a cylindrical plug having an orifice therethrough which can similarly be securely fitted to the metal nozzle so as to ensure safety of the surgical dissection instrument. The metal body is laser welded to the tube. Such a construction is used because it is believed by those in the art to be of primary importance to ensure that the orifice is accurately formed (as it can be in the metal plug) and most importantly that the metal plug is securely bonded into the nozzle bore. However, as for the use of a sapphire body, the use of a metal body inserted into the nozzle and laser welded thereto to provide the orifice adds expense and complexity to the manufacture of what is a disposable item. Again, if it is not completely ensured that the metal plug is permanently and securely bonded into the nozzle, the safety of the device is compromised. It can be difficult to fit the metal tube to the plastic conduit of the handpiece.

In addition, the surgical dissection instrument disclosed in EP-A-0840626 requires the rod of the nozzle to be rigid. This reduces the versatility of the surgical dissection instrument.

Other known surgical dissection instruments are disclosed in, for example, US-A-6508823, US-A-6423027, US-A-6423028, and US-A-63222533. US 4898574 discloses a lithotomic apparatus including a probe for insertion into the body cavity.

The present invention aims in one aspect to provide a nozzle for generating a pressure jet of liquid and a surgical dissection instrument comprising such a nozzle which ameliorates the problems of the known instruments discussed above.

The present invention aims in another aspect to provide a method of producing a nozzle for a surgical dissection instrument using a high-pressure liquid jet which ameliorates the problems of the known instruments discussed above.

According to the present invention there is provided a nozzle for use in generating a pressure jet of liquid in a surgical dissection instrument according to claim 1 wherein the nozzle has a proximal end for connection to a source of pressurised liquid and a distal end at which the pressure jet is generated, characterised in that the nozzle comprises a hollow tube having an axial lumen wherein the lumen has a restriction at the distal end in which an orifice is formed wherein the orifice has a width and an axial length, the width of the orifice being less than that of the lumen, and wherein the hollow tube and the restriction being integrally moulded from a plastic material.

The orifice preferably has an axis which is substantially parallel with the axis of the lumen. The axial length of the orifice of the nozzle is preferably such that the pressure jet of liquid generated by the nozzle is a surgical pressure jet of liquid. A surgical pressure jet of liquid is a pressure jet of liquid which is particularly suitable for use in surgery. It is preferably a well defined or coherent pressure jet having a length of from 5 to 7 cm (preferably about 6 cm) from the distal end of the nozzle before the pressure jet becomes substantially less well defined or coherent or it becomes a spray.

The nozzle preferably has an additional lumen which is preferably arranged such that the axes of the two lumen are substantially parallel wherein the additional lumen contains a stiffening element.
According to the invention there is provided a surgical dissection instrument for generating a pressure jet of liquid, the instrument comprising a handpiece, the handpiece being provided with an inlet conduit for receiving a supply of liquid to form a pressure jet and a nozzle according to the invention wherein the nozzle is in fluid communication with the inlet conduit and extends from the handpiece.

The instrument according to the invention preferably has a mechanism for controlling the pressure jet of liquid

The plastic material is preferably a plastics material having mechanical strength, purity, chemical resistance, ease of processing (including mouldability) and sterilization resistance. The plastic material is more preferably a thermoplastic polymeric material, especially a thermoplastic polycondensate. Examples of suitable plastic materials include a polyimide, polycarbonate, polyetheretherketone, polyaryletherketone, polyphenylene oxide, polysulfone and/or a polyphenylene sulphide. Most preferably the plastic material is a polyaryletherketone resin

According to the invention there is also provided a method of manufacturing a nozzle for a surgical dissection instrument according to claim 16 for generating a pressure jet of liquid, the method comprising the steps of:
extruding a plastic material to provide a hollow tube having an axial lumen;
moulding an end of the extruded hollow tube to form an integral restriction at the end; and
forming an axially directed orifice which extends through the restriction to the lumen, the width of the orifice being less than that of the lumen; and
including a stiffening element in the other of said two lumen.
The method of the invention preferably further comprises the step of cutting the tube to a selected length.

The present invention will now be disclosed in more detail with reference to the description of a preferred form of embodiment given by way of an unrestricted example and illustrated by the attached drawings, in which:
**Figure 1** shows an axial sectional view of a nozzle of a known surgical dissection instrument;
**Figure 2** shows an axial sectional view of a first embodiment of a hand-operated surgical dissection instrument not according to the present invention,
**Figure 3** shows an enlarged axial sectional view of the distal end of a first embodiment of a nozzle not according to the invention;

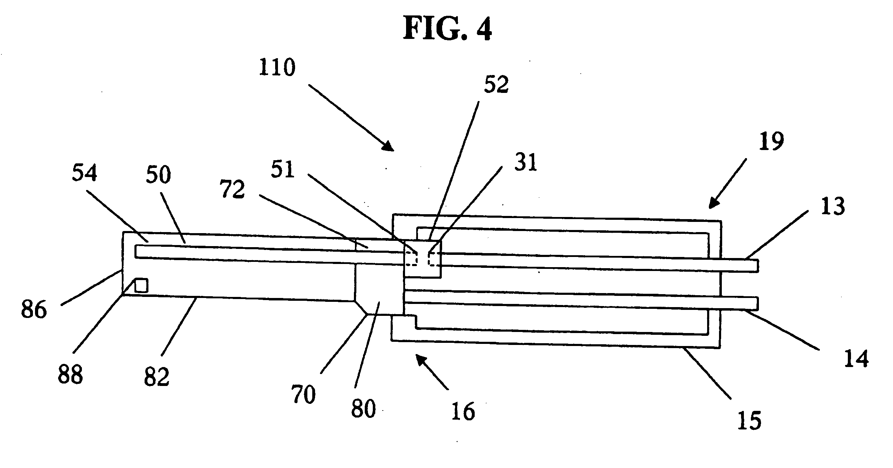

**Figure 4** shows an axial sectional view of a second embodiment of a hand-operated surgical dissection instrument not according to the present invention;
**Figure 5** shows an enlarged axial sectional view of the distal end of a nozzle according to the invention;
**Figure 6** shows an enlarged axial sectional view of the distal end of a nozzle according to the invention;
**Figure 7** shows an enlarged transverse sectional view of a nozzle according to the invention taken in the directions A-A' and B-B' shown in Figures 5 and 6, respectively; and
**Figure 8** shows an enlarged projection of the distal end of a nozzle of the invention as shown in Figure 5.

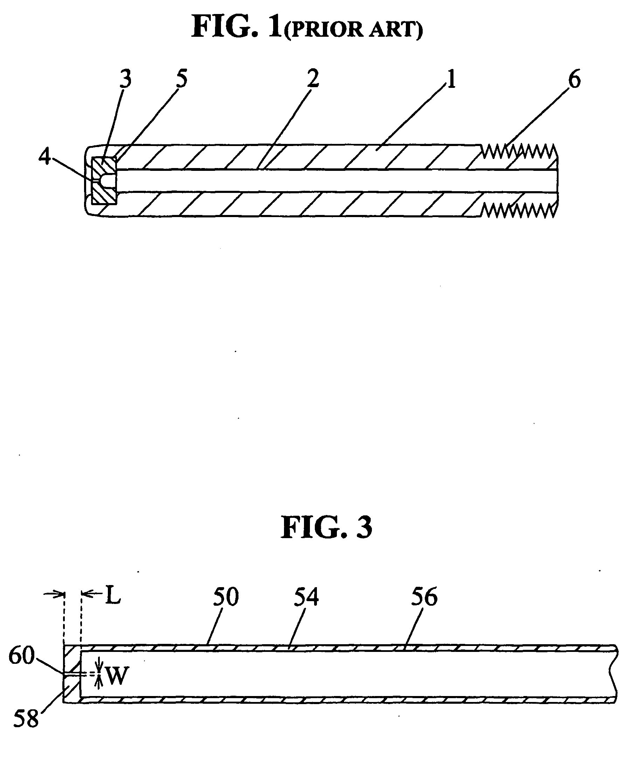

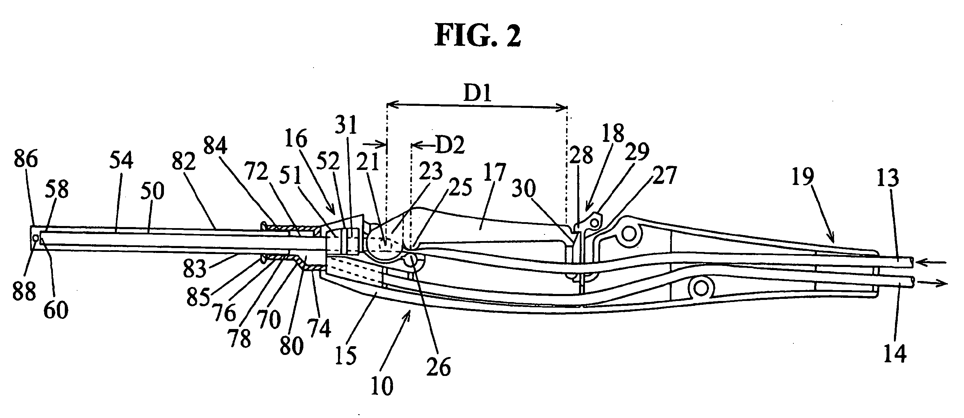

With reference to Figures 2 and 3, an embodiment of a surgical dissection instrument not according to the invention comprises a handpiece 10, a first flexible tube 13 for connection, in use, to a source (not shown) for supplying a high-pressure physiological salt solution and a second flexible tube 14 for connection, in use, to a suction device (not shown). The first flexible tube 13 is typically reinforced by a reinforcement made of braided synthetic wires, so as to be able to withstand the high pressure of the liquid, which may reach 70 bar.

The handpiece 10 is of the disposable type, made entirely from injection-moulded thermoplastic synthetic material. It primarily comprises an ergonomic body 15 of generally elongated shape, with a substantially circular cross-section, this body extending from a distal end 16 to a proximal end 19, both ends 16. 19 being substantially located on the same axis. The proximal end 19 incorporates an opening through which the tubes 13 and 14 enter the body 15.

The handpiece 10 furthermore comprises a control lever 17, including (as described below) means for pinching the flexible tube 13, and a device 18 for locking the control lever in the closed position in which the tube 13 is sealed and the jet of high-pressure liquid stopped.

The ergonomic body 15 is made up of two substantially identical moulded half shells. The two half shells are hollow on the inside to allow the tubes 13 and 14 to pass inside the body of the handpiece from the proximal end 19 to the distal end 16 along a substantially straight path.

The control lever 17 has an outer surface shaped to receive the user's fingers or palm. It is located in a central upper area of the ergonomic body 15 and articulated on a pivot 21 engaged in opposed openings (not shown) in the half shells of the ergonomic body 15 in an area close to the distal end 16. The pivot 21 is in fact comprised of two symmetrical elements each of which is an inwardly directed lug 23, both lugs 23 being arranged spaced from and parallel to one another. The spaced lugs 23 provide a passage (not shown) for the high-pressure physiological salt solution supply tube 13.

In an area extending proximal of the passage there is a movable squeezing projection 25 secured to the control lever 17, the movable squeezing projection 25 being positioned facing a fixed squeezing projection 26 secured to the body 15 of the handpiece and designed to cooperate with the latter to squash the tube 13, when the user presses the control lever 17, and instantaneously stop the flow of high-pressure liquid. More precisely, the squeezing projections 25, 26 define between them a space for the tube 13 to pass and they are arranged perpendicular to the tube 13. The movable squeezing projection 25 is designed to move in relation to the fixed squeezing projection 26 so as to reduce said space until it is cancelled thus ensuring that said tube 13 is fully squashed in a direction which is substantially perpendicular to the tube 13. The free end of the control lever 17 defines with the pivot 21 a lever arm D 1 and the movable squeezing projection 25 defines with said pivot 21 a lever arm D2 much smaller than D1. The ratio of the lever arms is preferably from 4 to 6. In the embodiment illustrated, the ratio of the lever arms D1, D2 is approximately 5. As a result, the operating force to be applied on the control lever 17 is divided by five. This original construction makes it possible to compress the tube 13 to a greater or lesser extent, even with a very high pressure, by operating the lever with your fingers easily, flexibly and without a large effort being required.

The locking means 18 are primarily comprised of a flexible tongue 27 secured to the ergonomic body 15 of the handpiece 10 and located in a substantially perpendicular plane to the free end of the control lever 17. The tongue 27 bears a catch pin 28 close to its upper end which is fitted with a pusher 29. The catch pin 28 is designed to cooperate with a grip 30 secured to the control lever 17. When the user presses the control lever 17 hard enough, this causes the flow of high-pressure physiological salt solution to stop by pinching the flexible tube 13. In order to release the lever 17, the user acts on the pusher 29, using his thumb, which has the effect of releasing the grip 30 from cooperation with the pin 28. The flexibility of the flexible tube 13 and the pressure of the physiological salt solution conveyed by this tube urge the control lever 17 back to its initial position in which the tube 13 is fully open.

The foregoing features of the handpiece 10 of the illustrated first embodiment of a hand-operated surgical dissection instrument of the present invention are substantially disclosed in EP-A-0840626 and its equivalent US-A-6066150.

The handpiece 110 of the illustrated second embodiment of a hand-operated surgical dissection instrument not according the present invention is a simplified version of the handpiece 10 of the first embodiment. Like features of the two embodiments are identified by like identification numerals. The handpiece 110 of the second embodiment of the invention is suitable for use with a source (not shown) of high pressure physiological salt solution which has means (not shown) for controlling the supply of the salt solution to the handpiece 110, e.g. a foot operated switch. Thus the control lever 17 and associated parts are superfluous in the second embodiment of the handpiece 110 and have been omitted.

In accordance with the present invention, an elongate nozzle 50 of plastic material is securely fitted to the first tube 13. The distal end 31 of first tube 13 and the proximal end 51 of the nozzle 50 are respectively securely fitted to a respective side of an annular collar 52 of plastic material, for example by push fitting the respective ends 31, 51 of the tube 13 and the nozzle 50 into respective annular ends of the collar 52 and then bonding the assembly with adhesive or by thermal bonding.

The nozzle 50 comprises a cylindrical tube 54 defining an axially directed central bore or lumen 56 extending therealong and having at its distal end an integral restriction 58. The restriction 58 is moulded from the plastic material of the tube 54 of the nozzle 50 so as to form a unitary and integral structure, without any connection or interface between the restriction 58 and the tube 54. An axially directed central orifice 60 extends through the restriction 58. The restriction 58 typically has an axial length (L) of from 0.1, preferably from 0.3mm to 2mm, preferably to 1 mm, more preferably to 0.5mm typically approximately 0.8 mm or about 0.4mm, with the orifice 60 having the same axial length. Thus the orifice is an elongated orifice. The diameter of the bore 56, and therefore the diameter of the proximal face of the restriction 58 subjected to liquid pressure in the bore 56 and the internal diameter of the tube 54, is typically from 1.5 to 2.5 mm, more typically approximately 1.7 mm. The internal diameter (W) of the orifice 60 is typically from 0.05 to 0.4 mm, more typically approximately 0.2 mm (e.g. about 0.22mm). The ratio of the axial length L to the internal diameter W is from 1:1, preferably from 1.2:1, more preferably from 1.3:1 to 5:1. preferably to 4.5:1. more preferably to 4:1. Preferably the ratio of L to W is about 1.5:1 or about 3.7:1. This structure of the restriction 56 is sufficient to withstand typical liquid pressures in the nozzle 50 of up to 70 bar without failure or deformation of the restriction 58.

The external diameter of the tube 54 is typically from 2 to 4 mm, more typically approximately 2.3 mm, thereby giving a typical wall thickness of from 0.5 to 2 mm, more typically approximately 0.6 mm. Such a wall thickness, together with the properties of the plastic material employed for the nozzle 50, results in the nozzle being sufficiently flexible that the nozzle 50 can readily be selectively disposed in a curved orientation under manual lateral pressure applied by the surgeon's hand. The nozzle 50 is sufficiently elastic for the nozzle 50 to return to a straight axially aligned configuration when the lateral pressure is removed. This results in a more versatile instrument than the known device which employs a rigid metal nozzle.

The nozzle 50 is typically composed of a thermoplastic polymeric material, especially a thermoplastic polycondensate such as a polyimide, polycarbonate, polyetheretherketone, polyaryletherketone, polyphenylene oxide, polysulfone and/or polyphenylene sulphide. Most preferably it is composed of a polyaryletherketone resin, which exhibits mechanical strength, purity, chemical resistance, ease of processing and sterilization resistance. This material can provide the flexibility, elasticity and resistance to high pressure liquid as discussed above.

The nozzle 50 is a one-piece construction made by the following process so as to form in the tube 54 an integral restriction 58 having the orifice 60 therethrough.

In the embodiment, the nozzle 50 is produced in two steps, step 1 being continuous extrusion of the plastic material through an extrusion die defining the outer diameter of the nozzle 50 and having a central mandrel defining the inner diameter of the nozzle 50, and step 2 being thermoforming of the end with a tip-forming machine using specific tooling for the external shape, the thermoforming also forming the orifice 60 by use of a correspondingly shaped and dimensioned pin of the tooling. The orifice may however be formed after the thermoforming step. Most preferably, the plastic material extrusion is extruded as a continuous length, and is cut into individual nozzle length pieces, the cutting being before or after the thermoforming step.

This manufacturing process provides the advantage over the known nozzles described hereinabove that it is not necessary to form two pieces (tube and sapphire needle or tube and metal plug) and then bond them together. A one piece construction is not only easier and less expensive to manufacture, but also it may have greater integrity than a two piece construction, because the internal fluid pressure cannot (as potentially for the known two piece constructions using sapphire/metal plug) cause the restriction part containing the orifice to break away and constitute a highly dangerous projectile under the high liquid pressures typically employed. A plastic tube of indeterminate length can be extruded, the required length can be severed off, and then the restriction can be integrally formed in the tube so that the nozzle has the required length. This readily provides nozzles of infinitely varying length. It is not necessary to form metal tubes having only a single length or a set of tubes having predetermined lengths.

Also, by using a plastic tube for the nozzle, this can readily be securely affixed to the plastic tube or collar in the handpiece for delivering the liquid to the nozzle. A plastic-plastic bond can be achieved by adhesive or thermal bonding. This obviates the need for a threaded coupling between the metal tube and the plastic tube or collar. Again, this avoids the previous need for predetermined nozzle lengths, and the cost of forming a thread. The coupling between the plastic tubes can be more reliably achieved than by using a threaded connection. This enhances the quality control of the instrument.

A manifold 70 is bonded in a fluid-tight manner to the distal end 16 of the ergonomic body 15. The manifold 70 surrounds the proximal portion 72 of the nozzle 50, the remainder of the nozzle 50 extending distally away from the manifold 70. The manifold 70 comprises a relatively large diameter proximal portion 74 attached to the distal end 16 and a relatively small diameter distal portion 76, defining an internal cylindrical surface 78. The manifold 70 defines an internal chamber 80 which is in fluid communication with the second flexible tube 14 which, in use, is connected to a source of suction.

An aspirator tube 82 of plastic material is fitted to the distal portion 76 of the manifold 70 by a proximal end 84 of the aspirator tube 82 being push fitted into the distal portion 76 so that the external surface 83 of the aspirator tube 82 mates with the internal cylindrical surface 78. An external annular stop ring 85 on the external surface 83 controls the depth that the aspirator tube 82 is push-fitted into the manifold 70. The aspirator tube 82 is cylindrical and surrounds the nozzle 50, with the nozzle 50 being substantially axially centrally located in the aspirator tube 82. The distal end 86 of the aspirator tube 82 is located a small distance. about 1 to 2 mm or less, distally of the distal end of the nozzle 50. Therefore the nozzle 50 is totally enclosed within the aspirator tube 82. The distal end 86 of the aspirator tube 82 is provided with at least one vent hole 88 extending through the wall thickness thereof. In the illustrated embodiment there are two diametrically opposed circular vent holes 88, each of a diameter of about 1 to 1.5 mm. The at least one vent hole 88 is provided to obviate the aspirator tube 82 from inadvertently attaching itself to the patient's body under the negative pressure applied to the aspirator tube 82.

The aspirator tube 82 is typically composed of polyvinylchloride and typically has a wall thickness of from 0.25 to 1.0 mm so as to be flexible together with the nozzle 50, with typically the inner diameter being from 3.5 to 5 mm and the outer diameter being from 4 to 6 mm. The aspirator tube 82 is preferably transparent so that a user can readily check that it has not become inadvertently blocked in use.

In a preferred embodiment, the nozzle and aspirator tube can be manufactured as an integral unit, constituting a double lumen, with an inner lumen and a, preferably coaxial, outer lumen. The nozzle and aspirator tube may be coextruded. The nozzle for the liquid jet constitutes the inner lumen and the aspirator tube constitutes the outer lumen. The outer lumen may be adapted to inject a drug; to cause articulation of the inner lumen and/or the outer lumen: to contain an electrode of an electrosurgery unit; to guide a laser beam therealong from a laser; and/or to guide an argon beam, or any of these in combination.

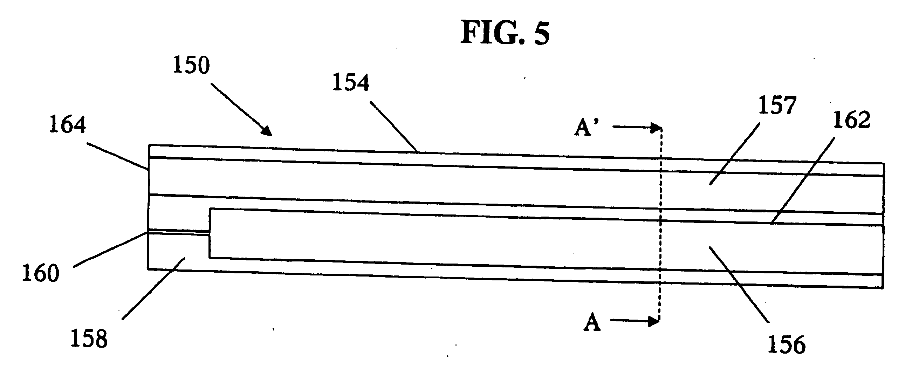

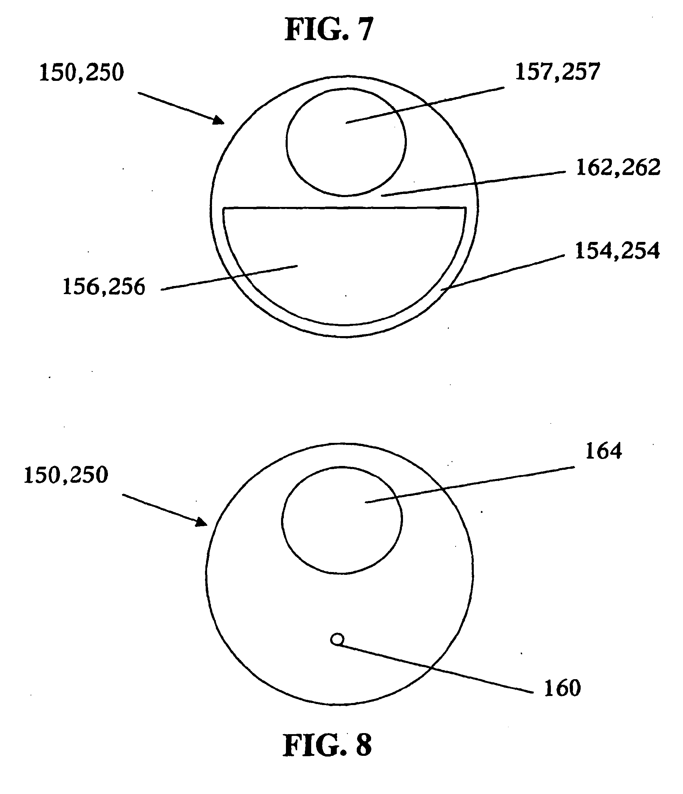

The embodiment of a nozzle 150 according to the present invention. is shown in Figure 5. Nozzle 150 is provided in the form of a cylindrical tube 154 having two substantially parallel lumen or bores 156,157 separated by a web 162. A cross-sectional view of the nozzle 150 taken along line A-A' is shown in Figure 7. Lumen 157 terminates at the distal end of the nozzle 150 with an orifice 164. Lumen 156 terminates at the distal end of the nozzle 150 with an integral restriction 158 having an axially directed central orifice 160 extending through it. An enlarged projection of the distal end of the nozzle 150 is shown in Figure 8. This Figure shows orifice 164 of lumen 157 and orifice 160 of lumen 156. The length of the integral restriction 158 and the width of the orifice 160 are substantially the same as the integral restriction 58 and orifice 60 for the first embodiment of the nozzle according to the invention. Lumen 157 is useful for the injection of a fluid; e.g. a medicament or for containing an electrode of an electrosurgery unit.

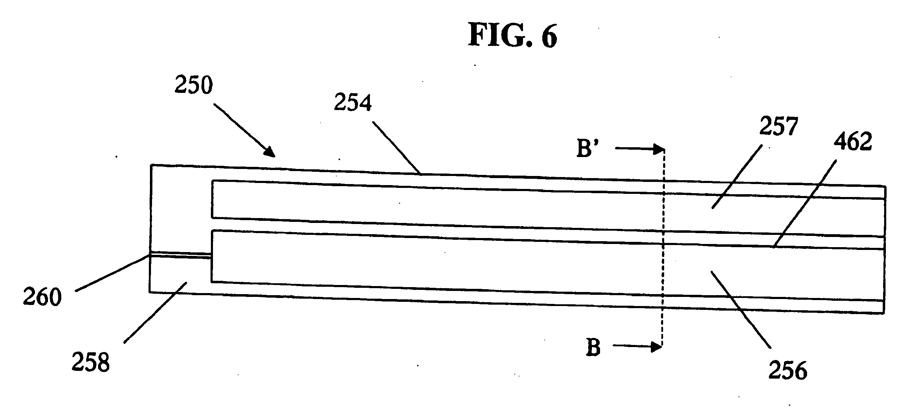

A third embodiment of a nozzle 250 according to the present invention is shown in Figure 6. Nozzle 250 is provided in the form of a cylindrical tube 254 having two substantially parallel lumen or bores 256,257 separated by a web 262. A cross-sectional view of the nozzle 250 taken along line B-B' is shown in Figure 7. Lumen 257 terminates at the distal end of the nozzle 250 with an integral restriction 258 such that the distal end of lumen 257 is closed. Lumen 256 terminates at the distal end of the nozzle 250 with an integral restriction 258 having an axially directed central orifice 260 extending through it. The length of the integral restriction 258 and the width of the orifice 260 are substantially the same as the integral restriction 58 and orifice 60 for the first embodiment of the nozzle according to the invention. Lumen 257 is useful for containing a stiffening element (not shown) such as a wire, particularly a metal wire. This is useful because it enables that the nozzle according to the third embodiment of the invention to be used in laparoscopic (or "key hole") surgery. In laparoscopic surgery, movement of the nozzle inside a patient's body is controlled from outside the patient's body. For this to work, the nozzle must not be flexible. Therefore, the stiffening element in lumen 257 is useful because it can provide sufficient stiffness for the nozzle to be used in this way.

The present invention is not restricted to the forms of embodiment described, but can undergo various alterations and be presented in various aspects derived from the forms described in an obvious manner for a person skilled in the art. For example, although the illustrated embodiment of the surgical dissection instrument for generating a pressure jet of liquid includes a handpiece incorporating a mechanical clamp for opening and closing the tube for supplying the liquid to form the jet, other handpiece constructions may be used, for example using a pneumatic control for the liquid to form the jet. Other types of handpiece suitable for use in the surgical dissection instrument of the invention are known to those skilled in the art and are encompassed within the scope of the present claims.

## Claims

1. A nozzle (150) for use in generating a pressure jet of liquid in a surgical dissection instrument wherein the nozzle has a proximal end for connection to a source of pressurised liquid and a distal end at which the pressure jet is generated, the nozzle comprising a flexible hollow tube (154) having an axial lumen (156) wherein the lumen has a restriction (158) at the distal end in which an orifice (160) is formed wherein the orifice (160) has a width and an axial length, the width of the orifice (160) being less than that of the lumen (156), and wherein the hollow tube (154) and the restriction (158) are integrally moulded from a flexible plastic material such that the nozzle is of one piece construction;
wherein the nozzle has an additional lumen (157) which is preferably arranged such that the axes of the two lumen (156, 157) are substantially parallel; and **characterised in that**
the additional lumen contains a stiffening element.

2. A nozzle as defined in Claim 1 wherein the axial length of the orifice (160) is such that the pressure jet of liquid generated by the nozzle is a surgical pressure jet of liquid.

3. A nozzle as defined in Claim 1 or Claim 2 wherein the ratio of the axial length of the orifice to its width is 1:1 to 5:1.

4. A nozzle as defined in any one of the preceding claims wherein the additional lumen (257) is closed at its distal end.

5. A nozzle as defined in any one of the preceding claims wherein the stiffening element is a wire, preferably a metal wire.

6. A nozzle as defined in any one of Claims 1 to 4, wherein the nozzle (150) is sufficiently flexible that the nozzle can be selectively disposed
in a curved orientation under manual lateral pressure applied by a surgeon's hand during dissection on a patient.

7. A nozzle as defined in Claim 6 which is sufficiently elastic for the nozzle to return to a straight axially aligned configuration when the lateral pressure is removed.

8. A nozzle as defined in any one of the preceding Claims which is composed of a thermoplastic polymeric material, preferably a thermoplastic polycondensate, especially a polyaryletherketone resin.

9. A surgical dissection instrument for generating a pressure jet of liquid, the instrument comprising a handpiece (10), the handpiece (10) being provided with an inlet conduit (13) for receiving a supply of liquid to form a pressure jet and a nozzle (150) as defined in any one of the preceding Claims
wherein the nozzle is in fluid communication with the inlet conduit (13) and extends from the handpiece (10).

10. An instrument as defined in Claim 9, which has a mechanism (17) for controlling the pressure jet of liquid.

11. An instrument as defined in Claim 9 or Claim 10, wherein the handpiece (10) further comprises an annular collar (52) of plastic material which connects the nozzle (150) to the inlet conduit (13), the respective ends of the hollow tube (154) and the inlet conduit (13) being push fitted into respective annular ends of the collar (52) and the push-fitted assembly being bonded with adhesive or by thermal bonding.

12. An instrument as defined in any one of Claims 9 to 11, wherein the handpiece (10) further comprises a manifold (70) at a distal end thereof, the manifold (70) surrounding a proximal portion of the nozzle (150) and defining an internal chamber (80) which is in fluid communication with a suction tube (82) which extends through the handpiece (10) and, in use, is connected to a source of suction.

13. An instrument as defined in Claim 12, wherein the handpiece (10) further comprises an aspirator tube (82) of plastic material fitted to a distal portion of the manifold (70), the aspirator tube (82) being cylindrical and surrounding the nozzle (150).

14. An instrument as defined in Claim 13, wherein the aspirator tube (82) has a wall thickness such that it is sufficiently flexible that the aspirator tube (82) can be selectively disposed in a curved orientation under manual lateral pressure applied by a surgeon's hand during dissection on a patient.

15. An instrument as defined in Claim 13 or Claim 14, wherein a proximal end of the aspirator tube (82) is push fitted into the distal portion of the manifold.

16. A method of manufacturing a flexible nozzle for a surgical dissection instrument for generating a pressure jet of liquid according to claim 1, the method comprising the steps of:
extruding a flexible plastic material to provide a hollow tube having two axial lumen:
moulding an end of the extruded hollow tube to form an integral restriction at the end; and
forming an axially directed orifice which extends through the restriction to one of the lumen, the width of the orifice being less than that of the lumen; and
including a stiffening element in the other of said two lumen.

17. A method as defined in Claim 16, further comprising the step of cutting the tube to a selected length.

18. A method as defined in Claim 16 or Claim 17, wherein the nozzle is sufficiently flexible that the nozzle can be selectively disposed in a curved orientation under manual lateral pressure applied by a surgeon's hand during dissection on a patient.

19. A method as defined in Claim 18, wherein the nozzle is sufficiently elastic for the nozzle to return to a straight axially aligned configuration when the lateral pressure is removed.

20. A method as defined in any one of Claims 16 to 19. wherein the plastic material is a thermoplastic polymeric material, preferably a thermoplastic polycondensate, especially a polyaryletherketone resin.

## Patentansprüche

1. Düse (150) zum Erzeugen eines Druckflüssigkeitsstrahls in einem chirurgischen Sezierelement, wobei die Düse ein proximales Ende zur Verbindung mit einer Druckflüssigkeitsquelle und ein distales Ende aufweist, an dem der Druckstrahl erzeugt wird, wobei die Düse ein flexibles hohles Rohr (154) mit einem axialen Lumen (156) aufweist,
wobei das Lumen an dem distalen Ende, in dem eine Öffnung (160) ausgebildet ist, eine Verengung (158) aufweist, wobei die Öffnung (160) eine Breite und eine axiale Länge aufweist, wobei die Breite der Öffnung (160) kleiner ist als diejenige des Lumens (156), und wobei das hohle Rohr (154) und die Verengung (158) integriert aus einem flexiblen Kunststoffmaterial geformt sind, so dass die Düse eine einstückige Konstruktion hat;
wobei die Düse ein zusätzliches Lumen (157) aufweist, das vorzugsweise derart angeordnet ist, dass die Achsen der beiden Lumina (156, 157) im Wesentlichen parallel sind; und
**dadurch gekennzeichnet, dass** das zusätzliche Lumen ein Versteifungselement enthält.

2. Düse nach Anspruch 1, bei der die Axiallänge der Öffnung (160) derart bemessen ist, dass der durch die Düse erzeugte Druckflüssigkeitsstrahl ein chirurgischer Druckflüssigkeitsstrahl ist.

3. Düse nach Anspruch 1 oder Anspruch 2, bei der das Verhältnis der Axiallänge der Öffnung zu ihrer Breite 1:1 bis 5:1 beträgt.

4. Düse nach einem der vorhergehenden Ansprüche, bei der das zusätzliche Lumen (257) an seinem distalen Ende geschlossen ist.

5. Düse nach einem der vorhergehenden Ansprüche, bei der das Versteifungselement ein Draht, vorzugsweise ein Metalldraht ist.

6. Düse nach einem der Ansprüche 1 bis 4, wobei die Düse (150) hinreichend flexibel ist, dass die Düse durch manuellen Seitendruck, der von der Hand eines Chirurgen während des an einem Patienten vorgenommenen Seziervorgangs aufgebracht wird, selektiv in einer gekrümmten Ausrichtung angeordnet werden kann.

7. Düse nach Anspruch 6, bei der die Düse hinreichend elastisch ist, dass sie in eine geradlinige, axial ausgerichtete Konfiguration zurückkehren kann, wenn der Seitendruck entfällt.

8. Düse nach einem der vorhergehenden Ansprüche, wobei die Düse aus einem thermoplastischen Polymermaterial ausgebildet ist, vorzugsweise einem thermoplastischen Polykondensat, insbesondere einem Polyaryletherketonharz.

9. Chirurgisches Sezierinstrument zum Erzeugen eines Druckflüssigkeitsstrahls, wobei das Instrument ein Handstück (10) aufweist, wobei das Handstück (10) mit einer Einlassleitung (13) zum Empfang einer zugeführten Flüssigkeit zwecks Bildung eines Druckstrahls und mit einer Düse (150) nach einem der vorhergehenden Ansprüche versehen ist, wobei die Düse in Fluidverbindung mit der Einlassleitung (13) steht und von dem Handstück (10) absteht.

10. Instrument nach Anspruch 9, mit einem Mechanismus (17) zum Steuern des Druckflüssigkeitsstrahls.

11. Instrument nach Anspruch 9 oder Anspruch 10, bei dem das Handstück (10) ferner einen ringförmigen Kragen (52) aus Plastikmaterial aufweist, der die Düse (150) mit der Einlassleitung (13) verbindet, wobei die betreffenden Enden des hohlen Rohrs (154) und der Einlassleitung (13) passend in betreffende ringförmige Enden des Kragens (52) gedrückt sind und die durch passendes Zusammendrücken gebildete Baugruppe mittels Kleber oder durch Wärmebonden verbondet wird.

12. Instrument nach einem der Ansprüche 9 bis 11, bei dem das Handstück (10) ferner an einem distalen Ende ein Sammelrohr (70) aufweist, wobei das Sammelrohr (70) ein proximales Ende der Düse (150) umgibt und eine Innenkammer (70) bildet, die in Fluidverbindung mit einem Saugrohr (82) steht, das durch das Handstück (10) verläuft und bei Betrieb mit einer Saugkraftquelle verbunden ist.

13. Instrument nach Anspruch 12, bei dem das Handstück (10) ferner ein Saugrohr (82) aus Kunststoffmaterial aufweist, dass passend an einem distalen Ende des Sammelrohrs (70) befestigt ist, wobei das Saugrohr (82) zylindrisch ist und die Düse (150) umgibt.

14. Instrument nach Anspruch 13, bei dem das Saugrohr (82) eine Wanddicke zur Erzielung einer hinreichenden Flexibilität dahingehend hat, dass das Saugrohr (82) durch manuellen Seitendruck, der von der Hand eines Chirurgen während des an einem Patienten vorgenommenen Seziervorgangs aufgebracht wird, selektiv in einer gekrümmten Ausrichtung angeordnet werden kann.

15. Instrument nach Anspruch 13 oder Anspruch 14, bei dem ein proximales Ende des Saugrohrs (82) in den distalen Teil des Sammelrohrs druckeingepasst ist.

16. Verfahren zur Herstellung einer gemäß Anspruch 1 ausgebildeten, für ein chirurgisches Sezierelement vorgesehenen flexiblen Düse zum Erzeugen eines Druckflüssigkeitsstrahls, mit den folgenden Schritten:
Extrudieren eines flexiblen Plastikmaterials zur Bildung eines hohlen Rohrs mit zwei axialen Lumina;
Formen eines Endes des extrudierten hohlen Rohrs zur Bildung einer integrierten Verengung an dem Ende; und
Formen einer axial ausgerichteten Öffnung, die durch die Verengung hindurch zu einem der Lumina verläuft, wobei die Breite der Öffnung kleiner ist als diejenige des Lumens; und
Einbeziehen eines Versteifungselements in das andere der beiden Lumina. '

17. Verfahren nach Anspruch 16, ferner mit dem Schritt des Schneidens des Rohrs auf eine gewählte Länge.

18. Verfahren nach Anspruch 16 oder Anspruch 17, bei dem die Düse hinreichend flexibel ist, dass die Düse durch manuellen Seitendruck, der von der Hand eines Chirurgen während des an einem Patienten vorgenommenen Seziervorgangs aufgebracht wird, selektiv in einer gekrümmten Ausrichtung angeordnet werden kann.

19. Verfahren nach Anspruch 18, bei dem die Düse hinreichend elastisch ist, dass sie in eine geradlinige, axial ausgerichtete Konfiguration zurückkehren kann, wenn der Seitendruck entfällt.

20. Verfahren nach einem der Ansprüche 16 bis 19, bei dem das Kunststoffmaterial ein thermoplastisches Polymermaterial, vorzugsweise ein thermoplastisches Polykondensat und insbesondere ein Polyaryletherketonharz ist.

## Revendications

1. Buse (150) destinée à être utilisée pour produire un jet de liquide sous pression dans un instrument chirurgical de dissection, dans laquelle la buse a une extrémité proximale pour le raccordement à une source de liquide pressurisé et une extrémité distale au niveau de laquelle est généré le jet sous pression, la buse comprenant un tube creux flexible (154) ayant un lumen axial (156), lequel possède un étranglement (158) au niveau de l'extrémité distale sur laquelle est formé un orifice (160),
dans laquelle l'orifice (160) a une largeur et une longueur axiale, la largeur de l'orifice (160) étant inférieure à celle du lumen (156), et dans laquelle le tube creux (154) et l'étranglement (158) sont moulés en un bloc à partir d'une matière plastique flexible de sorte que la buse soit d'une construction en une pièce ;
dans laquelle la buse a un lumen supplémentaire (157) qui est de préférence disposé de sorte que les axes des deux lumens (156, 157) soient essentiellement parallèles ; et **caractérisée en ce que**
le lumen supplémentaire contient un élément de rigidification.

2. Buse selon la revendication 1, dans laquelle la longueur axiale de l'orifice (160) est telle que le jet de liquide sous pression produit par la buse est un jet de liquide chirurgical sous pression.

3. Buse selon la revendication 1 ou la revendication 2, dans laquelle le rapport entre la longueur axiale de l'orifice et sa largeur est de 1/1 à 5/1.

4. Buse selon l'une quelconque des revendications précédentes, dans laquelle le lumen supplémentaire (257) est fermé à son extrémité distale.

5. Buse selon l'une quelconque des revendications précédentes, dans laquelle l'élément de rigidification est un fil, de préférence un fil métallique.

6. Buse selon l'une quelconque des revendications 1 à 4, dans laquelle la buse (150) est suffisamment flexible pour que la buse soit disposée sélectivement dans une orientation incurvée sous une pression latérale manuelle exercée par la main d'un chirurgien au cours de la dissection réalisée sur un patient.

7. Buse selon la revendication 6, qui est suffisamment élastique pour que la buse reprenne une configuration axialement alignée droite quand la pression latérale cesse.

8. Buse selon l'une quelconque des revendications précédentes, qui est constituée d'une matière polymère thermoplastique, de préférence un polycondensé thermoplastique, en particulier une résine de polyaryléther-cétone.

9. Instrument chirurgical de dissection pour produire un jet de liquide sous pression, l'instrument comprenant une pièce à main (10), la pièce à main (10) étant munie d'un conduit d'admission (13) destiné à recevoir une alimentation de liquide afin de former un jet sous pression et une buse (150) selon l'une quelconque des revendications précédentes, dans lequel la buse est en communication de fluide avec le conduit d'admission (13) et s'étend depuis la pièce à main (10).

10. Instrument selon la revendication 9, qui a un mécanisme (17) pour contrôler le jet de liquide sous pression.

11. Instrument selon la revendication 9 ou la revendication 10, dans lequel la pièce à main (10) comprend en outre une bague annulaire (52) en matière plastique qui raccorde la buse (150) au conduit d'admission (13), les extrémités respectives du tube creux (154) et du conduit d'admission (13) étant emmanchées par poussée dans les extrémités annulaires respectives de la bague (52) et l'assemblage emmanché étant lié par un adhésif ou par thermoliaison.

12. Instrument selon l'une quelconque des revendications 9 à 11, dans lequel la pièce à main (10) comprend en outre un collecteur (70) à une extrémité distale de celle-ci, le collecteur (70) entourant une partie proximale de la buse (150) et définissant une chambre interne (80) qui est en communication de fluide avec un tube d'aspiration (82) qui s'étend à travers la pièce à main (10) et, à l'usage, est raccordée à une source d'aspiration.

13. Instrument selon la revendication 12, dans lequel la pièce à main (10) comprend en outre un tube aspirateur (82) en matière plastique emmanché sur une partie distale du collecteur (70), le tube aspirateur (82) étant cylindrique et entourant la buse (150).

14. Instrument selon la revendication 13, dans lequel le tube aspirateur (82) a une épaisseur de paroi de manière à être suffisamment flexible pour que le tube aspirateur (82) puisse être sélectivement disposé dans une orientation incurvée sous une pression latérale manuelle exercée par la main d'un chirurgien au cours de la dissection réalisée sur un patient.

15. Instrument selon la revendication 13 ou la revendication 14, dans lequel une extrémité proximale du tube aspirateur (82) est emmanchée par poussée dans la partie distale du collecteur.

16. Procédé de fabrication d'une buse flexible destinée à un instrument chirurgical de dissection pour produire un jet de liquide sous pression selon la revendication 1, le procédé comprenant les étapes consistant à :
extruder une matière plastique flexible pour fournir un tube creux ayant deux lumens axiaux ;
mouler une extrémité du tube creux extrudé pour former un étranglement intégré à l'extrémité ; et
former un orifice dirigé axialement, qui s'étend à travers l'étranglement vers un des lumens, la largeur de l'orifice étant inférieure à celle du lumen ; et
comprenant un élément de rigidification dans l'autre desdits deux lumens.

17. Procédé selon la revendication 16, comprenant en outre l'étape consistant à couper le tube à une longueur choisie.

18. Procédé selon la revendication 16 ou la revendication 17, dans lequel la buse est suffisamment flexible pour que la buse puisse être disposée sélectivement dans une orientation incurvée sous la pression latérale manuelle exercée par la main d'un chirurgien au cours de la dissection réalisée sur un patient.

19. Procédé selon la revendication 18, dans lequel la buse est suffisamment élastique pour reprendre une configuration axialement alignée droite quand la pression latérale cesse.

20. Procédé selon l'une quelconque des revendications 16 à 19, dans lequel la matière plastique est une matière polymère thermoplastique, de préférence un polycondensé thermoplastique, en particulier une résine de polyaryléther-cétone.
